# EUROPEAN PATENT APPLICATION

(11) **EP 4 122 946 A1**
(43) Date of publication of application: **25.01.2023**
(21) Application number: 21767492.8
(22) Date of filing: 04.02.2021
(51) Int. Cl.: C07K 14/015, C12N 15/35, C12N 15/864, C12N 7/01, A61K 48/00, A61P 27/16

(54) **ADENO-ASSOCIATED VIRUS VECTOR AND USE THEREOF**

(30) Priority: 13.03.2020 CN 202010177739
(71) Applicant: Huigene Therapeutics Co., Ltd., Shanghai 200131 (CN)
(72) Inventor: YAO, Xuan, Shanghai 200131 (CN); SHI, Linyu, Shanghai 200131 (CN); WANG, Shaoran, Shanghai 200131 (CN); BAI, Weiya, Shanghai 200131 (CN)
(74) Representative: Marks & Clerk LLP
(86) International application number: PCT/CN2021/075213
(87) International publication number: WO 2021/179861

(57) **Abstract**

Provided are an AAV capsid protein mutant and an encoding nucleic acid, and a corresponding vector and a host cell thereof. Also provided are an adeno-associated virus vector containing the AAV capsid protein mutant, a recombinant adeno-associated virus particle constructed therefrom and carrying a gene expression cassette, a preparation method therefor, and the use thereof in treating diseases.

## Description

### Claim of Priority

The present application claims priority from Chinese patent applications No. 202010177739.5 filed on March 13, 2020, the contents of which are incorporated herein by reference in their entirety.

### Technical Field

The present invention relates to the field of genetic engineering and gene therapy, in particular, the present invention relates to an AAV capsid protein mutant, an adeno-associated virus (AAV) vector comprising the capsid protein mutant, an AAV virus particle constructed therefrom and carrying a gene expression cassette, a method for generating and using such an adeno-associated virus vector or virus particle, and the use of such an adeno-associated virus vector and virus particle in the treatment of diseases.

### Sequence Listing

The present application comprises a Sequence Listing, which is incorporated herein by reference.

### Background Art

The cochlea of the inner ear is the organ responsible for peripheral sound perception. Acoustic cells in cochlea play a very important role in the process of perceiving peripheral sound, and transform external acoustic wave signals into electrophysiological signals, and then gradually transmits the electrophysiological signals to the auditory center of brain through spiral ganglion cells of the inner ear.

Hair cell death caused by inherent hereditary or various acquired trauma can cause hearing impairment in varying degrees, even life-long deafness. According to the survey of the World Health Organization (WHO), 0.3% of neonates, 5% of population younger than 45 years old and 50% of population over 70 years old suffer from hearing impairment in varying degrees. Hearing impairment not only affects hearing itself, but also causes social disorder in varying degrees. Among them, hereditary deafness is caused by mutation of certain genes in the inner ear.

There are currently 100 known genes that cause deafness. Among them, GJB2 mutation is in the highest proportion and accounts for about 50% of hereditary deafness, and this gene is expressed in supporting cells; SLC26A4 gene mutation is in the second highest proportion and accounts for about 15% of hereditary deafness. Myo15A and OTOF genes are expressed in hair cells, and account for 5%-8% of hereditary deafness, respectively.

Gene therapy is the most promising therapy for treating hereditary diseases such as hearing loss, but it depends on the use of gene expression vectors. Studies have shown that the introduction efficiency of a gene into inner ear cells is low, which not only affects the study of inner ear gene function, but also hinders the gene therapy of hereditary deafness. Therefore, it is very important to screen vectors that can be efficiently transduced into inner ear cells, especially inner ear supporting cells.

Adeno-associated virus (AAV) has a broad application prospect in the field of human gene therapy; because of its long-term gene expression ability and no pathogenicity, AAV has been widely used in liver, muscle, heart, brain, eye, kidney and other tissues in various studies.

However, there are not much identified AAV vectors that can efficiently infect inner ear cells, especially inner ear supporting cells. Previous studies have found that the inner ear supporting cells are hardly infected with various serotypes of AAV, such as AAV1-AAV9; the inner ear supporting cells are also hardly infected with PHP.eB, a variant of AAV9; the supporting cells can be infected with another AAV: Anc80L65, and the infection efficiency is about 40%, but it is very difficult to obtain, and the virus yield is very low when the virus is enveloped; although the supporting cells can be infected with AAV2.7m8 virus, the infection efficiency is less than 20%, and higher titer is needed to achieve the infection efficiency of about 20%; and the supporting cells can be infected with AAV-DJ, which is easy to produce, but the ability thereof to infect the inner ear supporting cells needs to be improved.

Ubiquitin-proteasome degradation mechanism is considered as the main obstacle of AAV infection. Among them, point mutation of viral capsid protein (cap) at specific sites is the simplest and most common method, which can make viral vectors avoid phosphorylation, subsequent ubiquitination and proteasome-mediated degradation in cells. It has been documented that the point mutations of AAV2 and AAV8 viruses at specific tyrosine, serine, threonine or lysine can significantly improve the infection efficiency of vectors in liver (Nishanth Gabriel et al., HUMAN GENE THERAPY METHODS, 24: 80-93, 2013).

Adeno-associated virus (AAV) vector-mediated gene therapy has been approved in the United States for the treatment of rare hereditary ocular diseases and hereditary central nervous system diseases, but a safe and effective vector capable of targeting the inner ear cells has not been identified. Therefore, there is a need in the art for a novel AAV vector with high infection efficiency in the inner ear cells, especially in the inner ear supporting cells.

### Summary of the Invention

The present invention relates to a novel AAV capsid protein mutant, the amino acid sequence thereof is mutated compared with the amino acid sequence of parent or wild-type AAV capsid protein, so that the supporting cells of the inner ear of mammalian can be better targeted.

The present invention also relates to an AAV virus particle comprising the AAV capsid protein mutant, which is easy to produce, has a good safety and a high targeting, and has a high infection efficiency in the supporting cells (such as apical, middle and basal supporting cells) of the inner ear of mammal. The virus particle can be used for gene therapy of hearing disorders caused by gene defects in supporting cell. The AAV virus particle can directly carry defective genes or other therapeutic sequences and enter the inner ear supporting cells to express and exert the therapeutic effects; it can also enter the inner ear supporting cells to repair the defective genes in the genome, repair the transcripts of defective genes or provide a new and correct gene sequence by means of the carried gene editing tools therein.

Therefore, in one aspect, the present invention provides an AAV capsid protein mutant or a functional fragment thereof, wherein the AAV capsid protein mutant comprises an amino acid sequence having an amino acid mutation, as compared to an amino acid sequence of a parent or wild-type AAV-DJ capsid protein, at at least one position corresponding to positions 491, 500 and 666 in an amino acid sequence of a parent or wild-type AAV-DJ capsid protein set forth in SEQ ID NO: 1. In some embodiments, the present invention provides a AAV capsid protein mutant or a functional fragment thereof, wherein the AAV capsid protein mutant comprises an amino acid sequencehaving an amino acid mutation, as compared to the amino acid sequences of the parent or wild-type capsid proteins of AAV-DJ, AAV1, AAV2, AAV3A, AAV3B, AAV4, AAV5, AAV6, AAV7, AAV8, AAV9, AAV10, AAV11, AAV12, or AAV13, at one or more amino acid positions corresponding to positions 491, 500 and 666 of SEQ ID NO: 1. Optionally, the amino acid sequences of the parent or wild type capsid proteins of AAV1, AAV2, AAV3A, AAV3B, AAV4, AAV5, AAV6, AAV7, AAV8, AAV9, AAV10, AAV11, AAV12, or AAV13 are respectively set forth in SEQ ID NOs: 6-19.

In some embodiments, the functional fragment is a N-terminal truncated functional fragment of the AAV capsid protein mutant. In some specific embodiments, the functional fragment is a fragment with amino acid deletion at a position corresponding to positions 1-137 in an amino acid sequence of a parent or wild type AAV-DJ capsid protein set forth in SEQ ID NO: 1. In some other embodiments, the functional fragment is a fragment with amino acid deletion at a position corresponding to positions 1-202 in an amino acid sequence of a parent or wild type AAV-DJ capsid protein set forth in SEQ ID NO: 1.

In some embodiments, the amino acid mutation is one or more amino acid mutations selected from the group consisting of: an amino acid mutation at an amino acid position corresponding to positions 491, 500 and 666 of SEQ ID NO: 1. The mutation can be a deletion or substitution, wherein the substitution can be a substitution of serine with other amino acids, for example an amino acid that is not easily modified by phosphorylation, including for example, an acidic amino acid, a basic amino acid, a non-polar amino acid, or an aromatic amino acid except tyrosine, or a small amino acid except threonine. In some specific embodiments, the amino acid mutation is a substitution of serine with alanine.

In some particular embodiments, the viral capsid protein mutant comprises an amino acid sequence having one or more amino acid substitutions selected from the group consisting of a substitution at position 491 of serine (S) with alanine (A) (S491A), a substitution at position 500 of serine (S) with alanine (A) (S500A), and substitution at position 666 of serine (S) with alanine (A) (S666A) compared to the parent or wild-type AAV-DJ capsid protein as set forth in SEQ ID NO: 1.

In some exemplary embodiments, the serine (S) at position 491 is mutated to alanine (A) in the viral capsid protein mutant compared to the parent or wild-type AAV-DJ capsid protein as set forth in SEQ ID NO: 1, and the resulting amino acid sequence is set forth in SEQ ID NO: 2. In some exemplary embodiments, the serine (S) at position 500 is mutated to alanine (A) in the viral capsid protein mutant compared to the parent or wild-type AAV-DJ capsid protein as set forth in SEQ ID NO: 1, and the resulting amino acid sequence is set forth in SEQ ID NO: 3. In some exemplary embodiments, the serine (S) at position 666 is mutated to alanine (A) in the viral capsid protein mutant compared to the parent or wild-type AAV-DJ capsid protein as set forth in SEQ ID NO: 1, and the resulting amino acid sequence is set forth in SEQ ID NO: 4.

In some embodiments, the viral capsid protein mutant has the activity of promoting the infection of inner ear cells of mammal, especially supporting cells by an AAV mutant.

In another aspect, the present invention provides a recombinant adeno-associated virus particle (also known as a recombinant adeno-associated virus vector), comprising:
(a) a capsid protein mutant, the capsid protein mutant comprises an amino acid sequence having an amino acid mutation, as compared to an amino acid sequence of a corresponding parent or wild-type AAV capsid protein, at one or more amino acid positions corresponding to amino acid positions 491, 500 and 666 in an amino acid sequence of a parent or wild-type AAV-DJ capsid protein as set forth in SEQ ID NO: 1; and
(b) a heterologous polynucleotide encoding a heterologous gene product.

In some embodiments, the mutation is substitution, for example, substitution of serine with alanine.

In some embodiments, the heterologous polynucleotide has an AAV ITR sequence at 5'- and 3'-end, and the ITR sequence can be an intact or non-intact ITR sequence. In some embodiments, ITR is non-intact, for example, D or D', or trs or B or B', or C or C', or A or A' region, or any combination of the above regions is deleted.

In some embodiments, the viral capsid protein mutant comprises an amino acid sequence having one or more amino acid substitutions selected from the group consisting of a substitution at position 491 of serine (S) with alanine (A) (S491A), a substitution at position 500 of serine (S) with alanine (A) (S500A), and substitution at position 666 of serine (S) with alanine (A) (S666A) compared to the parent or wild-type AAV-DJ capsid protein as set forth in SEQ ID NO: 1.

In some exemplary embodiments, the serine (S) at position 491 is mutated to alanine (A) in the viral capsid protein mutant compared to the parent or wild-type AAV-DJ capsid protein as set forth in SEQ ID NO: 1, and the resulting amino acid sequence is set forth in SEQ ID NO: 2. In some exemplary embodiments, the serine (S) at position 500 is mutated to alanine (A) in the viral capsid protein mutant compared to the parent or wild-type AAV-DJ capsid protein as set forth in SEQ ID NO: 1, and the resulting amino acid sequence is set forth in SEQ ID NO: 3. In some exemplary embodiments, the serine (S) at position 666 is mutated to alanine (A) in the viral capsid protein mutant compared to the parent or wild-type AAV-DJ capsid protein as set forth in SEQ ID NO: 1, and the resulting amino acid sequence is set forth in SEQ ID NO: 4.

In some embodiments, the heterologous gene is at least one selected from: a hearing-related gene, a related gene for gene editing and a selectable marker gene. Specifically, the hearing-related gene can be a gene that is expressed in the hair cells and/or supporting cells of the inner ear of mammal, for example, at least one selected from the group consisting of: *GJB2, SLC26A4, MYO6, CLDN14, ESRRB, TPRRPN, GJB3, MYO15A, CDH23, 12SrRNA, MYO15A, OTOF, TMC1, CDH23, TMPRSS3, POU3F4, USH2A, MYO1F, MYO7A, TRIOBP, KCNQ4, ADGRV1, PAX3, MITF, OTOA, PDZD7, TECTA, GPR98, KCNQ1 and Brn4.*

In some embodiments, the related gene for gene editing is selected from a coding gene of a gene editing enzyme and a guide RNA (gRNA) targeting a specific site. For example, the gene editing enzyme can be at least one selected from: a meganuclease, a zinc finger nuclease, a transcription activator-like effector nuclease, a CRISPR-Cas system, a base editor and a prime editing system.

In another aspect, the present invention relates to a polynucleotide, which encodes the AAV capsid protein mutant or the functional fragment thereof disclosed herein. The polynucleotide can be a DNA sequence, RNA sequence, or a combination thereof. In some embodiments, the polynucleotide encodes the amino acid sequence set forth in SEQ ID NO: 2, 3 or 4 or the functional fragment thereof, for example, a N-terminal truncated fragment. The polynucleotide may have the corresponding amino acid codon substituted with a codon encoding alanine (e.g., GCC) at one or more positions corresponding to amino acid positions 491, 500 and 666, as compared to the nucleotide sequence encoding the parent or wild type AAV-DJ capsid protein as set forth in SEQ ID NO: 5.

In another aspect, the present invention relates to a vector or a nucleic acid construct, comprising the nucleic acid sequence encoding the AAV capsid protein mutant described herein. In some embodiments, the vector or nucleic acid construct is selected from an expression vector, a shuttle vector, and an integrative vector.

In another aspect, the present invention provides a host cell, and the host cell contains a vector or nucleic acid construct as described herein, or a polynucleotide as described herein is integrated into the genome of the host cell. The host cell can be a prokaryotic cell or a eukaryotic cell. In some embodiments, the prokaryotic cell is *Escherichia coli.* In some embodiments, the eukaryotic cell can be selected from the group consisting of a yeast cell, a plant cell, a mammal cell, for example, a human cell (such as an HEK293T cell), or a combination thereof.

In another aspect, the present invention relates to a pharmaceutical composition, comprising the recombinant adeno-associated virus particle (component i) as disclosed herein and a pharmaceutically acceptable carrier/excipient (component ii). In some embodiments, the component i comprises 0.1-99.9 wt%, preferably 10-99.9 wt%, more preferably 70-99 wt% of the total weight of the pharmaceutical composition. In some embodiments, the pharmaceutical composition is formulated into a liquid preparation, for example, the dosage form is an injection. In some embodiments, the pharmaceutical composition is formulated into an injection for intracochlear injection. In some embodiments, the component ii is an excipient for injection, preferably, an excipient selected from the group consisting of a physiological saline, a glucose saline, or a combination thereof.

In one aspect, the present invention provides a method for infecting inner ear cells of a mammal subject, comprising administering the recombinant adeno-associated virus particle as disclosed herein to the mammal subject. In some embodiments, the inner ear cells are supporting cells. In some embodiments, the inner ear cells administered to the subject are optionally administered by injection, for example, intracochlear injection. In the present invention, the mammal is a human, monkey, ape, chimpanzee, cat, dog, bovine, horse, mouse, rat, rabbit, *etc.*

In one aspect, the present invention provides the use of the recombinant adeno-associated virus particle as disclosed herein as a drug.

In one aspect, the present invention provides the use of the recombinant adeno-associated virus particle as disclosed herein in the alleviation, improvement or treatment of the ear diseases such as a hearing disorder in a subject. In some embodiments, the subject is a patient having a hearing disorder. The patient can be a human or other mammals. The hearing disorder can be selected from the group consisting of: a hereditary deafness, a non-hereditary deafness, or a combination thereof, wherein the hereditary deafness can include deafness caused by a factor selected from the group consisting of gene mutation, gene deletion, or a combination thereof; the non-hereditary deafness can include deafness caused by a factor selected from the group consisting of: use of a drug, trauma, infection, aging, or any combination thereof. The subject can be a patient having hereditary deafness or a patient having non-hereditary deafness. In some embodiments, the subject is a patient having a hearing disorder caused by gene mutation in the inner ear supporting cells.

In some embodiments, the recombinant adeno-associated virus particle may be used alone, or for example, in combination with other drugs for treating hearing disorders. The other drugs for treating hearing disorders include but are not limited to an anti-infection antibiotic drug, a neurotrophic factor drug, an ion channel modulator drug, a vitamin drug and the like, or a combination thereof.

In another aspect, the present invention provides a method for treating ear diseases such as hearing disorders, comprising administering to a subject in need thereof the recombinant adeno-associated virus particle or the pharmaceutical composition as described herein. The manner of administration is preferably intracochlear injection. In some embodiments, the cause of the hearing disorder is mutation in hearing-related genes expressed in inner ear supporting cells.

In one aspect, the present invention provides a method for preparing the gene expression vector described herein, comprising operatively linking an expression cassette of a therapeutic gene for treating hearing disorders into the genome of an AAV vector, thereby obtaining the recombinant AAV vector as described herein.

In one aspect, the present invention provides a method for infecting hearing-related cells, comprising infecting the hearing-related cells with the recombinant AAV mutant described herein, wherein the recombinant AAV mutant contains a viral capsid protein mutant as described in the first aspect of the present invention. In some embodiments, the hearing-related cells are inner ear supporting cells, for example apical, middle or basal parts.

The foregoing is a summary and thus comprises, by necessity, simplifications, generalization, and omissions of detail; therefore, those skilled in the art will recognize that the summary is only for illustrative purposes and not intended to be limiting in any way. Other aspects, features and advantages of the method, composition and use and/or other subjects described herein will become apparent from the teaching set forth herein.

### Brief Description of the Drawings

Figure 1A shows a screening experimental design for supporting cell infection in mice by means of using AAV-DJ and a mutant thereof respectively. Figure 1B shows the structure of the AAV-rep/cap mutant plasmid in a three-plasmid system.
Figures 2A-2C show the results of the infection of supporting cells of apical part (A), middle part (B) and basal (C) part of mouse cochlea by AAV-DJ and a mutant thereof, respectively. "DJ" represents the parent AAV-DJ, and S491A, S500A and S666A respectively represent the corresponding 3 AAV-DJ mutants.
Figure 3 shows the results of statistical analysis of infection of supporting cells of mouse cochlea by AAV-DJ and a mutant thereof.

### Detailed Description of Embodiments

The present invention relates to an AAV vector capable of efficiently infecting inner ear supporting cells and the use thereof in gene therapy. Specifically, the present inventors engineer the cap sequence of a parent or wild-type AAV-DJ (for example, mutation, preferably substitution, of a specific serine site) to obtain an AAV vector capable of significantly improving the infection efficiency in inner ear supporting cells. The AAV vector can be used for constructing a gene expression vector carrying various heterologous polynucleotides (i.e., a recombinant AAV vector) for a variety of purposes. In addition, the AAV vector or the gene expression vector can be formulated together with a pharmaceutically acceptable carrier/excipient into a pharmaceutical composition.

### AAV Virus Comprising a Capsid Protein Mutant

Adeno-associated virus (AAV) belongs to the genus *Dependovirus* of the *Parvoviridae* family. The AAV viral particle is composed of two parts: first, icosahedral-like protein capsid, and a single-stranded viral genome DNA enveloped in the capsid. The genome of AAV is a single-stranded DNA molecule with a length of about 4.7-6 kb; and the genome comprises an inverted terminal repeat (ITR) of about 145 bp on both ends which can form a T-shaped hairpin structure and contains cis-acting elements required for DNA replication initiation and viral particle packaging. There are two open reading frames (ORF) in the middle of the genome, which are successively Rep gene and Cap gene from left to right. Rep gene encodes 4 non-structural proteins Rep78, Rep68, Rep52 and Rep40, which are mainly responsible for viral genome replication, transcriptional regulation, site-specific integration, *etc.* Cap gene encodes 3 structural proteins VP1, VP2 and VP3, which are transcribed by p40 promoter and constitute the capsid of AAV. Single AAV viral particle contains 60 copies of the three structural proteins (the ratio of VP1 : VP2 : VP3 is about 1 : 1 : 10). VP1 is necessary for the formation of infectious AAV; VP2 assists VP3 to enter the nucleus; VP3 is the major protein of AAV particle.

As used herein, the term "AAV" may be used to refer to a naturally occurring parent or wild type virus itself or a derivative thereof. Unless otherwise specified, the term covers all forms of subtype, serotype and pseudotype, as well as naturally occurring and recombinant forms. As used herein, the term "serotype" refers to AAVs identified on the basis of capsid protein reactivity with a given antiserum and distinguished from other AAVs, such as eight serotypes of AAVs present in primates, AAV1 to AAV8. By way of example, serotype AAV2 is used to refer to that the capsid of a virus is composed of a capsid protein encoded by the cap gene of AAV2.

There are various serotypes of AAV in nature, such as AAV2, 3, 5 and 6 isolated from human cells, and AAV1, 4 and 7-12 isolated from non-human animal cells, and more than 100 types of AAV mutants have been isolated from nature. The capsid proteins of different serotypes of AAV recognize different receptors on the cell surface, so the efficiency of infection on different tissues and cells is greatly different, showing a certain organ-targeting specificity. Using this feature, specific and efficient transduction of specific serotypes of AAV into specific types of cells and tissues can be achieved.

Current research has shown that the tissue tropism and cell transformation efficiency of AAV are mainly determined by the capsid thereof. Different types of capsids determine that different AAVs have different tissue tropism and transformation efficiency. Especially, amino acids in the triplex axis region of capsid are closely related to the binding of AAV to cell surface receptors. Minor changes in amino acids in the region may also lead to a significant change in tropism or transformation efficiency (Wu, Asokan et al., J Virol. 2006; 80 (22): 11393-11397; Excoffon, Koerber et al., Proc Natl Acad Sci USA. 2009; 106 (10): 3865-3870). Minor changes in some amino acids in other regions of AAV capsid may significantly affect the packaging ability of the virus (Bleker, Pawlita et al., J Virol. 2006; 80 (2): 810-820). Therefore, AAV vectors with high transduction efficiency and different infection characteristics can be screened by directed mutation engineering of Cap gene of AAV capsid protein by artificial evolution, which greatly increases the application potential of AAV.

AAV-DJ has a new synthesized AAV capsid protein (recombinant from AAV2, AAV8 and AAV9), and compared with other serotypes of AAV, AAV-DJ has higher transduction efficiency in various cell lines. In the present invention, in order to screen out an AAV vector capable of efficiently infecting inner ear cells, the Cap gene sequence of AAV-DJ (encoding an amino acid sequence as set forth in SEQ ID NO: 1 or a functional fragment thereof) is genetically engineered.

Thereof, in one aspect, the present invention provides an AAV capsid protein mutant or a functional fragment thereof, wherein the AAV capsid protein mutant comprises an amino acid sequence having an amino acid mutation, as compared to an amino acid sequence of an AAV parent or wild-type capsid protein, at one or more amino acid positions corresponding to positions 491, 500 and 666 in an amino acid sequence of a parent or wild-type AAV capsid protein as set forth in SEQ ID NO: 1. In some embodiments, the parent or wild-type AAV capsid protein has an amino acid sequence set forth in SEQ ID NO: 1 or an amino acid sequence having at least 85%, at least 90%, at least 91%, at least 92%, at least 93%, at least 94%, at least 95%, at least 96%, at least 97%, at least 98% or at least 99% sequence identity thereto.

In other embodiments, the parent or wild-type AAV capsid protein is from AAV1, AAV2, AAV3A, AAV3B, AAV4, AAV5, AAV6, AAV7, AAV8, AAV9, AAV10, AAV1 1, AAV12, or AAV13. For example, the parent or wild-type AAV capsid protein has an amino acid sequence selected from SEQ ID NOs: 6-19 or an amino acid sequence having at least 85%, at least 90%, at least 91%, at least 92%, at least 93%, at least 94%, at least 95%, at least 96%, at least 97%, at least 98% or at least 99% sequence identity thereto. In the present invention, sequence alignment, determination of the percentage of amino acid sequence identity and corresponding sequence positions can be performed according to some software known in the art, for example, BLAST program, CLUSTALW (http://www.ebi.ac.uk/clustalw/), MULTALIN (http://prodes.toulouse.inra.fr/multalin/cgi-bin/multalin.pl) or MUSCLE (Multiple Sequence Alignment), and the obtained sequences are probed (for example, aligned) by using default parameters indicated on the websites. As used herein, when used with respect to a particular pair of aligned amino acid sequences, the term "identity" or "percentage identity" refers to the percentage of amino acid sequence identity, which is obtained by means of counting the number of identical matches in the alignment and dividing such number of identical matches by the length of the aligned sequence.

For example, identity can be calculated by the Needleman-Wunsch global alignment and scoring algorithm (Needleman and Wunsch (1970) J. Mol. Biol. 48 (3): 443-453), as implemented by a "pin" program distributed as part of the EMBOSS package (Rice, P. et al. (2000) EMBOSS: The European Molecular Biology Open Software Suite, Trends in Genetics 16 (6): 276-277, and, among other resources, version 6.3.1 available from EMBnet at embnet.org/resource/emboss and emboss.sourceforge.net), using the default gap penalty and scoring matrix (EBLOSUM62 for protein and EDNAFULL for DNA). Any other similar sequence comparison program can also be used.

Additional mathematical algorithms are known in the art and can be used to compare two sequences. See, for example, algorithms in Karlin and Altschul (1990) Proc. Natl. Acad. Sci. USA 87:2264, and as modified in Karlin and Altschul (1993) Proc. Natl. Acad. Sci. USA 90: 5873-5877. The algorithm is introduced into the BLAST program of Altschul et al. (1990) J. Mol. Biol. 215:403. BLAST protein searches can be implemented by means of using the BLASTP program (a protein query for protein sequence searches) to obtain an amino acid sequence homologous to the AAV capsid protein. To obtain gapped alignments for comparison purposes, Gapped BLAST (in BLAST 2.0) can be utilized as described in Altschul et al. (1997) Nucleic Acids Res. 25:3389. Alternatively, PSI-Blast can be used for implementing an iterated search that detects distant relationships between molecules. See Altschul *et al.* (1997), *ibid.* When using BLAST, Gapped BLAST, and PSI-Blast programs, the default parameters of the corresponding programs, such as BLASTX and BLASTN, can be used. Alignment can also be implemented manually by means of checking.

When two sequences are aligned for similarity scores by means of using a defined amino acid substitution matrix (for example, BLOSUM62), gap existence penalty and gap extension penalty to obtain the highest possible score for the pair of sequences, they are "optimally aligned". Amino acid substitution matrix and the use thereof in the quantification of similarity between two sequences are well known in the art and are described, for example, in Dayhoff et al. (1978), "A model of evolutionary change in proteins." In "Atlas of Protein Sequence and Structure," Vol. 5, Suppl. 3 (edited by M. O. Dayhoff), pp. 345-352. Natl. Biomed. Res. Found., Washington, D.C. and Henikoff et al. (1992) Proc. Natl. Acad. Sci. USA 89:10915-10919. The BLOSUM62 matrix is often used as the default scoring substitution matrix in a sequence alignment scheme. A gap existence penalty is imposed when introducing a single amino acid gap into one of the aligned sequences, and a gap extension penalty is imposed for each additional empty amino acid position inserted into an already open gap. The alignment is determined by means of aligning the amino acid positions that begin and end on each sequence, and optionally by means of inserting a gap or gaps into one or both sequences in order to achieve the highest possible score. Although the optimal alignment and scoring can be manually completed, the process is facilitated by means of using a computer-implemented alignment algorithm, such as gap BLAST2.0, as described in Altschul et al. (1997) Nucleic Acids Res. 25: 3389-3402, which is publicly available on the National Center for Biotechnology Information Website (www.ncbi.nlm.nih.gov). Optimal alignments (including multiple alignments) can be prepared by means of using, for example, PSI-BLAST, and PSI-BLAST is available on www.ncbi.nlm.nih.gov and as described in Altschul et al. (1997) Nucleic Acids Res. 25: 3389-3402.

With regard to the amino acid sequence optimally aligned with the reference sequence, the amino acid residue "corresponds" to the position in the reference sequence to which the residue is paired in the alignment. "Position" is indicated by a number that sequentially identifies each amino acid in the reference sequence on the basis of the position thereof relative to the N-terminal. Due to deletions, insertions, truncations, fusions, and the like that must be taken into account when determining an optimal alignment, in general the amino acid residue number in a test sequence determined by simply counting from the N-terminal will not necessarily be the same as the number of its corresponding position in the reference sequence. For example, in the case where there is a deletion in an aligned test sequence, there is no amino acid at the deletion site corresponding to the position in the reference sequence. Where there is an insertion in an aligned reference sequence, the insertion will not correspond to any amino acid position in the reference sequence. In the case of truncations or fusions, there can be segments of amino acids in either the reference sequence or the aligned sequence that do not correspond to any amino acids in the corresponding sequence.

In some embodiments, the present invention provides an AAV capsid protein mutant comprising an amino acid sequence having a mutation at at least one serine site, as compared to the amino acid sequence of an AAV parent or wild type capsid protein as set forth in SEQ ID NO: 1. The capsid protein mutant can improve the infection efficiency of AAV virus vector in inner ear cells of mammals compared to the parent or wild type capsid protein.

As used herein, "amino acid mutation" refers to a change in the amino acid sequence of a predetermined amino acid sequence. Exemplary mutation includes addition, substitution and deletion. "Amino acid substitution" refers to that the existing amino acid residue in a predetermined amino acid sequence is substituted with another different amino acid residue. The substituted residues may be naturally occurring or non-naturally occurring amino acid residues. Preferably, in the present invention, the serine site in the amino acid sequence of the parent or wild-type AAV-DJ capsid protein is mutated.

"Amino acid addition" refers to that one or more amino acid residues are introduced into a predetermined amino acid sequence. Amino acid addition may comprise "peptide addition", in which case a peptide comprising two or more amino acid residues linked by peptide bonds is introduced into a predetermined amino acid sequence. If amino acid insertion involves the addition of peptide, the added peptide can be generated by random mutagenesis, so that it has an amino acid sequence that does not occur in nature.

In some exemplary embodiments, the serine site for mutation includes at least one selected from: S491, S500 and S666 (position numbering refers to SEQ ID NO: 1). The mutation may be substitution, deletion, addition, *etc.* of amino acids. Preferably, the mutation can be a substitution of serine with any other amino acids, for example an amino acid that is not easily modified by phosphorylation, for example, an acidic amino acid, a basic amino acid, a non-polar amino acid, or an aromatic amino acid except tyrosine, or a small amino acid except threonine. More preferably, the mutation is substitution of serine with alanine.

In some specific embodiments, the present invention provides an AAV capsid protein mutant comprising an amino acid sequence having S491A substitution or a functional fragment thereof, as compared to the amino acid sequence of a parent or wild-type AAV-DJ capsid protein as set forth in SEQ ID NO: 1. For example, the capsid protein mutant may comprise the amino acid sequence set forth in SEQ ID NO: 2 or consist of SEQ ID NO: 2; the functional fragment may be an N-terminal truncated functional fragment, for example, a functional fragment with an amino acid deletion at positions 1-137 or an amino acid deletion at positions 1-202.

In some specific embodiments, the present invention provides an AAV capsid protein mutant comprising an amino acid sequence having S500A substitution or a functional fragment thereof, as compared to the amino acid sequence of a parent or wild-type AAV-DJ capsid protein as set forth in SEQ ID NO: 1. For example, the capsid protein mutant may comprise the amino acid sequence set forth in SEQ ID NO: 3 or consist of SEQ ID NO: 3; the functional fragment may be an N-terminal truncated functional fragment, for example, a functional fragment with an amino acid deletion at positions 1-137 or an amino acid deletion at positions 1-202.

In some specific embodiments, the present invention provides an AAV capsid protein mutant comprising an amino acid sequence having S666A substitution or a functional fragment thereof, as compared to the amino acid sequence of a parent or wild-type AAV-DJ capsid protein as set forth in SEQ ID NO: 1. For example, the capsid protein mutant may comprise the amino acid sequence set forth in SEQ ID NO: 4 or consist of SEQ ID NO: 4; the functional fragment may be an N-terminal truncated functional fragment, for example, a functional fragment with an amino acid deletion at positions 1-137 or an amino acid deletion at positions 1-202.

As used herein, "functional fragment" refers to a portion of a polypeptide molecule (for example, a capsid protein) that contains at least 80%, 90%, 95%, or more of the full length of the polypeptide (for example, SEQ ID NOs: 2, 3, or 4) and retains the function of the full-length polypeptide. That is, the functional fragment described in the present invention can be successfully assembled into an AAV virus vector, and the infection efficiency of the AAV virus vector in inner ear cells of mammals can also be significantly improved compared with the original AAV-DJ capsid protein. In some embodiments, the functional fragment may be an N-terminal truncated functional fragment of SEQ ID NOs: 1, 2, 3 or 4, for example, a functional fragment with an amino acid deletion at positions 1-137 or an amino acid deletion at positions 1-202.

The capsid protein mutant in the present invention is not limited to the AAV-DJ capsid protein mutant and may be any of AAV1, AAV2, AAV3A, AAV3B, AAV4, AAV5, AAV6, AAV7, AAV8, AAV9, AAV10, AAV11, AAV12, AAV13 parent or wild-type capsid protein (SEQ ID NOs: 6-19, respectively) or any mutant thereof. Similarly, the mutant has an amino acid mutation, such as an amino acid deletion or substitution, at one or more positions corresponding to amino acid positions 491, 500 and 666 of the parent or wild-type AAV-DJ capsid protein as set forth in SEQ ID NO: 1.

The amino acid positions in the amino acid sequences of capsid proteins of different serotypes of AAV corresponding to the amino acid positions 491, 500 and 666 of AAV-DJ capsid protein amino acid sequence are summarized in Table A below.

**Table A. Sequence alignment results completed by MUSCLE (multiple sequence alignment)**

| AAV | Corresponding positions of amino acids in capsid protein | | | Source of amino acid sequence of AAV capsid protein |
|---|---|---|---|---|
| AAV-DJ | 491S | 500S | 666S | - |
| AAV1 | 490S | 499S | - | https://www.ncbi.nlm.nih.gov/nuccore/NC_002077 |
| AAV2 | 489S | 498S | - | https://www.ncbi.nlm.nih.gov/nuccore/NC_001401 |
| AAV-3A | 490S | 499S | - | https://www.ncbi.nlm.nih.gov/nuccore/545731199 |
| AAV-3B | 490S | 499S | - | https://www.ncbi.nlm.nih.gov/nuccore/AF028705.1 |
| AAV4 | 484S | 498S | - | https://www.ncbi.nlm.nih.gov/nuccore/NC_001829 |
| AAV5 | - | 485S | - | https://www.ncbi.nlm.nih.gov/nuccore/NC_006152 |
| AAV6 | 490S | 499S | - | https://www.ncbi.nlm.nih.gov/nuccore/AF028704.1 |
| AAV7 | 492S | 501S | - | https://www.ncbi.nlm.nih.gov/nuccore/NC_006260 |
| AAV8 | 492S | 501S | 667S | https://www.ncbi.nlm.nih.gov/nuccore/NC_006261 |
| AAV9 | 490S | 499S | - | https://www.ncbi.nlm.nih.gov/nuccore/AY530579.1 |
| AAV10 | 492S | 501S | - | https://www.ncbi.nlm.nih.gov/protein/?term=txid235456[Organism:noexp] |
| AAV11 | 483S | - | - | https://www.ncbi.nlm.nih.gov/protein/?term=txid235457[Organism:noexp] |
| AAV12 | 492S | - | - | https://www.ncbi.nlm.nih.gov/protein/?term=txid235458[Oranism:noexp] |
| AAV13 | 487S | 496S | - | https://www.ncbi.nlm.nih.gov/protein/?term=txid501327[Oreanism:noexp] |

Therefore, in some embodiments, the present invention provides an AAV capsid protein mutant comprising an amino acid sequence having an amino acid mutation, as compared to a corresponding amino acid sequence of a parent or wild-type AAV1 capsid protein as set forth in SEQ ID NO: 6, at one or more amino acid positions corresponding to positions 490 and 499 in an amino acid sequence of a parent or wild-type AAV1 capsid protein.

In some embodiments, the present invention provides an AAV capsid protein mutant comprising an amino acid sequence having an amino acid mutation, as compared to a corresponding amino acid sequence of a parent or wild-type AAV2 capsid protein as set forth in SEQ ID NO: 7, at one or more amino acid positions corresponding to positions 489 and 498 in an amino acid sequence of a parent or wild-type AAV2 capsid protein.

In some embodiments, the present invention provides an AAV capsid protein mutant comprising an amino acid sequence having an amino acid mutation, as compared to a corresponding amino acid sequence of a parent or wild-type AAV3A capsid protein as set forth in SEQ ID NO: 8, at one or more amino acid positions corresponding to positions 490 and 499 in an amino acid sequence of a parent or wild-type AAV3A capsid protein.

In some embodiments, the present invention provides an AAV capsid protein mutant comprising an amino acid sequence having an amino acid mutation, as compared to a corresponding amino acid sequence of a parent or wild-type AAV3B capsid protein as set forth in SEQ ID NO: 9, at one or more amino acid positions corresponding to positions 490 and 499 in an amino acid sequence of a parent or wild-type AAV3B capsid protein.

In some embodiments, the present invention provides an AAV capsid protein mutant comprising an amino acid sequence having an amino acid mutation, as compared to a corresponding amino acid sequence of a parent or wild-type AAV4 capsid protein as set forth in SEQ ID NO: 10, at one or more amino acid positions corresponding to positions 484 and 498 in an amino acid sequence of a parent or wild-type AAV4 capsid protein.

In some embodiments, the present invention provides an AAV capsid protein mutant comprising an amino acid sequence having an amino acid mutation, as compared to a corresponding amino acid sequence of a parent or wild-type AAV5 capsid protein as set forth in SEQ ID NO: 11, at an amino acid position corresponding to position 485 in an amino acid sequence of a parent or wild-type AAV5 capsid protein.

In some embodiments, the present invention provides an AAV capsid protein mutant comprising an amino acid sequence having an amino acid mutation, as compared to a corresponding amino acid sequence of a parent or wild-type AAV6 capsid protein as set forth in SEQ ID NO: 12, at one or more amino acid positions corresponding to positions 490 and 499 in an amino acid sequence of a parent or wild-type AAV6 capsid protein.

In some embodiments, the present invention provides an AAV capsid protein mutant comprising an amino acid sequence having an amino acid mutation as compared to a corresponding amino acid sequence of a parent or wild-type AAV7 capsid protein as set forth in SEQ ID NO: 13, at one or more amino acid positions corresponding to positions 492 and 501 in an amino acid sequence of a parent or wild-type AAV7 capsid protein,.

In some embodiments, the present invention provides an AAV capsid protein mutant comprising an amino acid sequence having an amino acid mutation, as compared to a corresponding amino acid sequence of a parent or wild-type AAV8 capsid protein as set forth in SEQ ID NO: 14, at one or more amino acid positions corresponding to positions 492, 501 and 667 in an amino acid sequence of a parent or wild-type AAV8 capsid protein.

In some embodiments, the present invention provides an AAV capsid protein mutant comprising an amino acid sequence having an amino acid mutation, as compared to a corresponding amino acid sequence of a parent or wild-type AAV9 capsid protein as set forth in SEQ ID NO: 15, at one or more amino acid positions corresponding to positions 490 and 499 in an amino acid sequence of a parent or wild-type AAV9 capsid protein.

In some embodiments, the present invention provides an AAV capsid protein mutant comprising an amino acid sequence having an amino acid mutation, as compared to a corresponding amino acid sequence of a parent or wild-type AAV 10 capsid protein as set forth in SEQ ID NO: 16, at one or more amino acid positions corresponding to positions 492 and 501 in an amino acid sequence of a parent or wild-type AAV10 capsid protein.

In some embodiments, the present invention provides an AAV capsid protein mutant comprising an amino acid sequence having an amino acid mutation, as compared to a corresponding amino acid sequence of a parent or wild-type AAV11 capsid protein as set forth in SEQ ID NO: 17, at an amino acid position corresponding to position 483 in an amino acid sequence of a parent or wild-type AAV11 capsid protein.

In some embodiments, the present invention provides an AAV capsid protein mutant comprising an amino acid sequence having an amino acid mutation, as compared to a corresponding amino acid sequence of a parent or wild-type AAV12 capsid protein as set forth in SEQ ID NO: 18, at an amino acid position corresponding to position 492 in an amino acid sequence of a parent or wild-type AAV12 capsid protein.

In some embodiments, the present invention provides an AAV capsid protein mutant comprising an amino acid sequence having an amino acid mutation, as compared to a corresponding amino acid sequence of a parent or wild-type AAV 13 capsid protein as set forth in SEQ ID NO: 19, at one or more amino acid positions corresponding to positions 487 and 496 in an amino acid sequence of a parent or wild-type AAV13 capsid protein.

Similarly, the mutation can be deletion or substitution. The substitution can be a substitution of serine with other amino acids, for example an amino acid that is not easily modified by phosphorylation, for example, an acidic amino acid, a basic amino acid, a non-polar amino acid, or an aromatic amino acid except tyrosine, or a small amino acid except threonine. The classification of amino acids is known in the art, wherein: basic amino acids include arginine, lysine and histidine; acid amino acids include glutamic acid and aspartic acid; polar amino acids include glutamine and asparagine; hydrophobic amino acids include leucine, isoleucine and valine; aromatic amino acids include phenylalanine and tryptophan; and small amino acids include glycine, alanine, serine and methionine.

In some specific embodiments, the amino acid mutation is a substitution of serine with alanine. In some specific embodiments, serine groups at positions 491, 500 and 666 of the amino acid sequences of the capsid protein mutant disclosed in the present invention are respectively substituted with alanine.

In addition, the present invention also encompasses any combination of mutations at positions 491, 500 and/or 666 of the amino acid sequences of the capsid protein mutant, that is, the mutation is not limited to a single-position mutation, but may occur at two or three amino acid positions. For example, mutations occur at amino acid positions 491 and 500, 500 and 666, 491 and 666, or 491, 500 and 666.

### Recombinant AAV vector (i.e., AAV gene expression vector)

Since AAV has not been found to be related to any human diseases, AAV virus can be engineered into an efficient exogenous gene transfer tool, that is, used as a gene expression vector. To date, no significant side effects related to AAV vectors have been found in all clinical trials using AAV vectors as gene introduction tools. Therefore, AAV vector has become one of the safest vectors for gene therapy and gene vaccine.

As used herein, the term "recombinant AAV vector", "recombinant AAV", "recombinant AAV virus", "recombinant AAV virus particle", "recombinant AAV virus particle" or "AAV gene expression vector" can be used interchangeably herein, which means that the genome DNA encapsulated in the AAV virus capsid contains a heterologous nucleic acid. Rep and Cap genes in the original genome can be eliminated in the vector, and replaced with heterologous polynucleotides expressing target genes, and the genetic substance elements carried by the vector can be expressed in host cells by infection, transformation, transduction or transfection into the host cells. In addition to a therapeutic gene and/or a reporter gene, the vector may comprise a plurality of elements for controlling expression, including but not limited to a promoter sequence, a transcription initiation sequence, an enhancer sequence, an intron, a kozak sequence, a polyA sequence and a selection element. Moreover, the vector may also comprise an origin of replication.

The assembly of recombinant AAV virus must rely on three components, namely: a transfer vector, which is composed of transgenic expression reading frame and ITR region of AAV at both flanking sides (which can be intact ITR or non-intact ITR); cap and rep coding sequences of AAV; and the function of helper virus. AAV vector has a relatively mature assembly system, which is convenient for large-scale production. At present, the common AAV vector assembly systems at home and abroad mainly include a three-plasmid co-transfection system, a system in which adenovirus is used as a helper virus, a packaging system in which herpes simplex virus is used as helper virus and a baculovirus-based packaging system. Among them, the three-plasmid transfection assembly system is the most widely used AAV vector assembly system because of its high safety, and is also the mainstream production system in the worldwide at present. Specifically, the three-plasmid transfection assembly system generally comprises three types of plasmids: a recombinant AAV transfer plasmid, in which only ITR sequences at both ends of the original genome (which can be intact ITR or non-intact ITR) are retained, and Rep and Cap genes of a parent or wild-type AAV are eliminated and replaced with heterologous polynucleotides expressing target genes; a recombinant AAV helper plasmid, which is a cloned genome of a parent or wild-type AAV with ITR deleted, and provides the function of a protein encoded by Rep and Cap genes in a trans manner; and an AdV helper virus plasmid comprising helper genes E2a, E4orf6 and VA RNA. E1a and E1b55k may be provided by the cells to be transfected, such as HEK293 cells.

Heterologous polynucleotides (also referred to as "polynucleotides of interest") described herein may encode various gene products (also referred to as "gene products of interest") for different therapeutic uses. Typically, such heterologous polynucleotides are located within the recombinant AAV vector and are flanked with an inverted terminal repeat (ITR) region. Various heterologous polynucleotides may be used in the present invention to produce recombinant AAV vectors for various different applications. Such heterologous polynucleotides include but are not limited to, for example, (i) polynucleotides suitable for gene therapy to mitigate defects caused by deletions, defects or suboptimal content of structural or functional proteins, such as polynucleotides encoding genes related to hearing defects; (ii) polynucleotides transcribed into antisense molecules; (iii) polynucleotides transcribed into baits binding to transcription or translation factors; (iv) polynucleotides encoding cell modulators, such as cytokines; (v) polynucleotides that sensitize recipient cells to specific drugs; (vi) polynucleotides for cancer therapy, such as E1A tumor suppressor gene or p53 tumor suppressor gene for treating various cancers; and (vii) polynucleotides encoding gene editing tools. The polynucleotide can be operatively linked to a promoter (its own or heterologous promoter) in order to achieve the expression of the gene product of interest in the recipient host cell. Many suitable promoters are known in the art, the selection thereof depends on the level of expression of the desired polynucleotide of interest; and whether constitutive expression, induced expression, cell-specific or tissue-specific expression, *etc.* is needed. The recombinant AAV vector may also contain selectable markers.

In the field of gene therapy, numerous drugs for gene therapy on the basis of the AAV vector have been developed, for example, Glybera, a drug for gene therapy for lipoprotein lipase on the basis of AAV vector; Luxturna, a drug for gene therapy for congenital amaurosis; Zolgensma, a drug for gene therapy for spinal muscular atrophy, *etc.,* all of which have achieved good clinical trial results.

### Recombinant AAV vector/gene expression vector for hearing disorders

Previous studies have shown that the efficiency of AAV virus vector for introducing a gene into inner ear cells is low, which not only affects the study of inner ear gene function, but also hinders the gene therapy of hereditary deafness. Therefore, it is very important to screen AAV vectors that can be efficiently transduced into inner ears.

Both hair cells (HC) and supporting cells (SC) in the inner ear can be used as targets for gene therapy. There are two types of sensory HC: an external HC (OHC), which amplifies sound, and an internal HC (IHC), which converts acoustic wave into electrical signals. The SC is located between the internal and external HC, anchors the sensory epithelium to the basal layer, protects the HC and maintains its environment. Half of sensorineural hearing loss is caused by gene mutations of HC and SC. Some key deafness genes are mainly expressed in SC and have functions, such as GJB2, which affects the gap junction of SC and is the most common gene responsible for hereditary deafness.

In the present invention, various AAV-DJ capsid protein mutants are respectively designed, packaged into AAV-DJ vectors expressing tdTomato, and injected into the cochlea of mice. Immunofluorescence analysis result of cochlear cells three weeks after injection shows that AAV-DJ vector comprising AAV-DJ capsid protein mutant containing S491A, S500A and/or S666A substitution can significantly improve the infection efficiency in inner ear supporting cells. This provides a tremendous help for the study of gene function and gene therapy strategies for supporting cells. Of course, the AAV capsid protein mutant of the present invention is not limited to AAV-DJ capsid protein mutant, as described above, it encompasses AAV1, AAV2, AAV3A, AAV3B, AAV4, AAV5, AAV6, AAV7, AAV8, AAV9, AAV10, AAV1 1, AAV12, AAV13 capsid proteins or any one of any mutants thereof having an amino acid mutation, such as deletion or substitution, preferably a substitution of serine with alanine, at one or more positions corresponding to amino acid positions 491, 500 and 666 of parent or wild type AAV-DJ capsid protein as set forth in SEQ ID NO: 1.

Genes of interest for the prevention, improvement, or treatment of hearing disorders include but are not limited to, hearing-related genes expressed in normal individuals (i.e., hearing-related genes of parent or wild type), and/or related genes for gene editing. The hearing-related gene can be a gene that is expressed in the inner ear supporting cells, for example, at least one selected from the group consisting of: *GJB2, SLC26A4, MYO6, CLDN14, ESRRB, TRPN, GJB3, MYO15A, CDH23, 12SrRNA, MYO15A, OTOF, TMC1, CDH23, TMPRSS3, POU3F4, USH2A, MYO1F, MYO7A, TRIOBP, KCNQ4, ADGRV1, PAX3, MITF, OTOA, PDZD7, TECTA, GPR98, KCNQ1, Brn4, etc.,* or any combination thereof; The related genes for gene editing may be at least one selected from the group consisting of: a gene editing enzyme (including a meganuclease, a zinc finger nuclease, a transcription activator-like effector nuclease, a CRISPR-Cas system, a base editor, a prime editing system, *etc.*) and a guide RNA (gRNA) targeting a specific site.

### Pharmaceutical composition

The present invention also relates to a pharmaceutical composition, comprising: (i) the recombinant AAV vector or the recombinant AAV virus particle as described herein; and (ii) a pharmaceutically acceptable carrier and/or an excipient.

As used herein, the term "pharmaceutically acceptable" refers to that the carrier, diluent, excipient, and/or a salt thereof are/is chemically and/or physically compatible with other ingredients in the formulation, and are/is physiologically compatible with the recipient.

As used herein, the term "pharmaceutically acceptable carrier and/or excipient" refers to a carrier and/or excipient that are/is pharmacologically and/or physiologically compatible with the subject and the active agent and that are/is well-known in the art (see, e.g., Remington's Pharmaceutical Sciences. Edited by Gennaro AR, 19th ed. Pennsylvania: Mack Publishing Company, 1995) and includes but is not limited to pH adjusting agents, surfactants, adjuvants, and ion strength enhancers. For example, the pH adjusting agents include but are not limited to phosphate buffer; the surfactants include but are not limited to cationic, anionic or nonionic surfactants, such as Tween-80; and the ion strength enhancers include but are not limited to sodium chloride. Pharmaceutically acceptable carriers can include such as pharmaceutically acceptable liquid, gel or solid carriers, aqueous media, non-aqueous media, antimicrobial agents, isotonic agents, buffering agents, antioxidants, anesthetics, suspending agents/dispersants, chelating agents, diluents, adjuvants, excipients or non-toxic auxiliary substances, a combination of various components known in the art or more.

In some embodiments, the recombinant AAV vector or recombinant AAV virus particle comprises 0.1-99.9 wt%, preferably 10-99.9 wt%, more preferably 70-99 wt% of the total weight of the pharmaceutical composition.

In some embodiments, the pharmaceutical composition is formulated into a liquid preparation, for example, an injection. In some embodiments, the pharmaceutical composition is formulated into an injection for intracochlear injection. In some embodiments, the component (ii) is a carrier for injection, preferably, one or more selected from the group consisting of: a physiological saline, a glucose saline, or a combination thereof.

The pharmaceutical composition of the present invention can be used for treating various diseases of mammal subjects such as ear diseases and more specifically, hearing disorders. The hearing disorder can be selected from the group consisting of: a hereditary deafness, a non-hereditary deafness, or a combination thereof, wherein, the hereditary deafness can include deafness caused by a factor selected from the group consisting of: gene mutation, gene deletion, or a combination thereof; the non-hereditary deafness can include deafness caused by a factor selected from the group consisting of: use of a drug, trauma, infection, aging, or any combination thereof. The subject can be a patient having hereditary deafness or a patient having non-hereditary deafness. In some embodiments, the subject is a patient having a hearing disorder caused by gene mutation in the inner ear supporting cells.

Administration to a mammal subject can be performed by a number of ways, including for example systemic or topical injection or infusion such as intravenous injection or infusion, and in particular intracochlear injection or infusion.

### Method of treatment

The present invention also provides a method for treating a ear disease of mammals, comprising administering the recombinant AAV vector or recombinant AAV virus particle of the present invention to the mammals. In some specific embodiments, the recombinant AAV vector or recombinant AAV virus particle is administered by systemic or topical injection or infusion, for example administered by intravenous injection or infusion, in particular by intracochlear injection or infusion. The ear disease may be for example, a hearing disorder. Preferably, the hearing disorder can be selected from the group consisting of: a hereditary deafness, a non-hereditary deafness, or a combination thereof, wherein, the hereditary deafness can include deafness caused by a factor selected from the group consisting of: gene mutation, gene deletion, or a combination thereof; the non-hereditary deafness can include deafness caused by a factor selected from the group consisting of: use of a drug, trauma, infection, aging, or any combination thereof. The subject can be a patient having hereditary deafness or a patient having non-hereditary deafness. In some embodiments, the subject is a patient having a hearing disorder caused by gene mutation in the inner ear supporting cells.

### Beneficial Effects

In some aspects, the AAV vector or AAV virus particle comprising an AAV-DJ capsid protein mutant disclosed herein provides the following beneficial effects:
1) high efficiency: The AAV vector or AAV virus particle of the present invention has high infection efficiency on inner ear supporting cells (especially basal supporting cells).
2) Easy production: The AAV vector or the AAV virus particle of the present invention has high virus yield and high stability, and it is easy to obtain the AAV viral particle with high titer and high quality in the production process.
3) Good safety: AAV is a vector approved by FDA for clinical treatment, and the AAV vector of the present invention has no damage to inner ear tissues.
4) High targeting: Compared with small molecule drugs, the AAV vector or AAV virus particle of the present invention has the characteristics of tissue and cell specific infection, and can target specific cell types.

### Examples

The present invention, thus generally described, will be understood more readily by reference to the following Examples, which are provided by way of illustration and are not intended to be limiting of the present invention. The Examples are not intended to represent that the experiments below are all or the only experiments performed.

### Experimental Materials and Methods

### Mouse

ICR mice (P1, purchased from Shanghai Lingchang Biotechnology Co., Ltd.) were used for AAV virus injection. The use and care of animals were done under the guidance of the Animal Ethics Committee.

### Cell culture and infection

HEK293T cells were cultured on a 10% FBS medium comprising the following ingredients: DMEM (Gibco, 11965-02), 10% fetal bovine serum (FBS) (Gibco), 2 mM glutamine (Gibco), 1% penicillin/streptomycin (Thermo Fisher Scientific) and 0.1 mM non-essential amino acid (Gibco). All cells were cultured at 5% CO₂ and 37°C.

The HEK293T cells were infected with AAV for 48 hours, then the expression of tdTomato was observed by a fluorescence microscope.

### AAV virus packaging

The 293T cells were transfected with a three-plasmid system comprising an AAV-rep/cap mutant plasmid (see Example 1 for specific construction), a pHelper plasmid (obtained from Institute of Neuroscience, Chinese Academy of Sciences) and an AAV-transgenic plasmid comprising a tdTomato fluorescent marker gene (obtained from Institute of Neuroscience, Chinese Academy of Sciences) for 4-6 h, and then the buffer was changed. The supernatant and cells were collected on the fourth day. The supernatant was precipitated with PEG overnight, then centrifuged at 4200 rpm at 4° C for 30 min, followed by centrifuged at 4400 rpm for another 10 min, and the supernatant was discarded. The precipitate was dissolve with 1xGB. The cells were repeatedly frozen and thawed with liquid nitrogen for three times. The supernatant and cells were digested with benzonase and 5 M NaCl for 30 min. After the digestion was completed, the mixture was centrifuged at 3000 g for 10 min, and the supernatant was taken. Density gradient ultracentrifugation was carried out at 68000 rpm at 18°C for 1 h 25 min. The layer was taken, diluted with PBS, and then concentrated with an ultrafiltration tube.

### AAV virus injection

ICR P0 mice, either gender, were randomly grouped according to different AAV mutants, with 4 mice in each group. The skin was cut at 2 mm from the posterior auricular sulcus with ophthalmic scissors under a stereo microscope, and the subcutaneous tissue was slightly separated. Facial nerve, posterior wall of auditory vesicle and posterior abdomen of digastric muscles could be seen. The lateral wall ligament of cochlea was punctured with a glass microelectrode, and 1 microliter virus was injected into cochlea of a mouse. After injection, the glass electrode was gently pull out and the incision was closed. 3 weeks after injection, samples were taken to analyze phenotype.

### Immunostaining analysis

In immunostaining experiment, mice were anesthetized with sodium pentobarbital (50 mg/Kg, Sigma), and then subjected to cardiac perfusion with 0.9% physiological saline and 4% paraformaldehyde through a peristaltic pump (Gilson), and then fixed overnight in 4% paraformaldehyde at 4°C. The next day, the tissues were decalcified in 10% EDTA. After decalcification, the basal membrane was separated under an anatomical microscope, and cut into three segments (apical, middle and basal parts). The separated basal membrane was washed three times with 0.1 M phosphate buffer (PB), and then incubated with primary antibody diluted with 5% NGS at 4°C overnight. The next day, the slices were washed three times with PB, and then incubated with secondary antibody at room temperature for two hours on a rotary shaking table. The slices finally were re-stained with DAPI for 20 minutes, and sealed with SlowFade Diamond AntifadeMountant (Life) on the slide.

### Antibody

Supporting cells: primary antibody: goat anti-Sox2 (Santa Cruz Biotechnology, sc-17320); secondary antibody: Alexa Fluor^{®} 488 AffiniPure donkey anti-goat IgG (H+L) (Jackson Immuno Research, 705-545-003)

Hair cells: primary antibody: rabbit anti-myosin VI polyclonal (Proteus Biosciences, 25-6791); secondary antibody: Cy^{™}5 AffiniPure donkey anti-rabbit IgG (H+L) (Jackson Immuno Research, 711-175-152)

### Data statistical analysis and software

The infection efficiency in supporting cells and hair cells was quantified, and the infection efficiency was explained by means of counting the proportion of mCherry+ cells in supporting cells within random 100 microns visual field. Results were obtained from at least 3 mice, and represented by mean ± standard deviation. *P < 0.05, ^{∗∗∗}P < 0.001, unpaired T test.
Snapgene: Used for plasmid profile construction design
Excel: Raw data processing
ImageJ: Experimental figure processing
Adobe photoshop CS6: Experimental figure processing
Adobe Illustrator CS4: Experimental figure processing
Example 1: Screening experimental design for AAV-DJ mutants

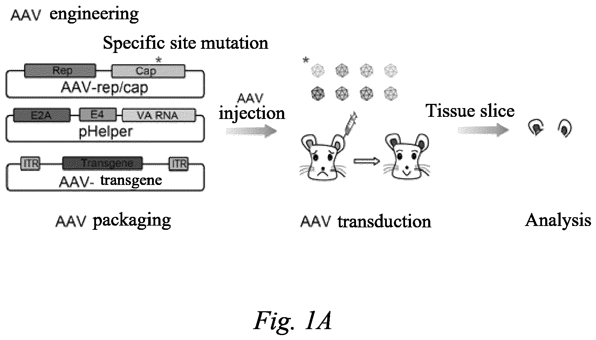

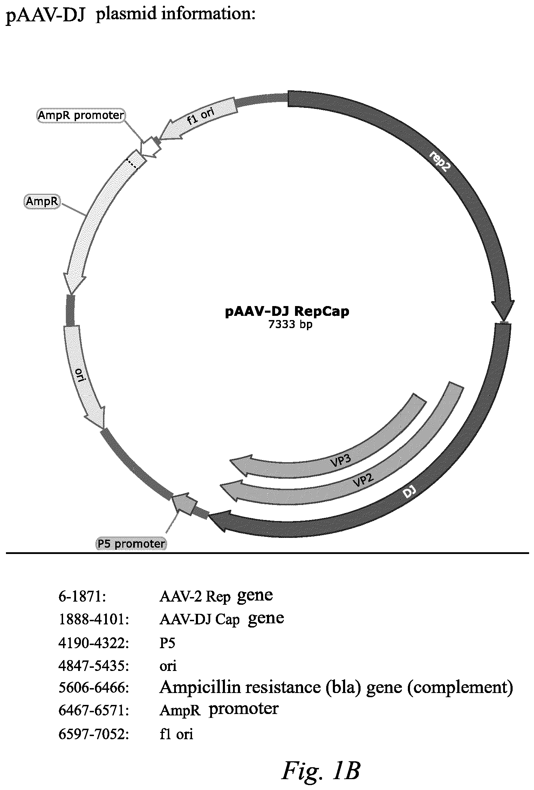

The cap sequence (SEQ ID NO: 1) of AAV-DJ (obtained from Institute of Neuroscience, Chinese Academy of Sciences, see Grimm D, et al., J Virol. 2008 Jun; 82 (12): 5887-911) was genetically engineered to screen an AAV vector that can efficiently infect supporting cells. Specifically, the inventors designed 3 types of AAV-DJ capsid protein mutants comprising the following substitutions, respectively: S491A, S500A, and S666A; the corresponding polynucleotides were obtained by PCR amplification (the alanines at the mutation sites were all encoded by GCC, and the nucleotides at the other sites were the same as the AAV-DJ cap gene sequence), and then the corresponding AAV-rep/cap mutant plasmids were constructed (see Figure 1B). The virus particles of AAV-DJ and its mutants (3 types) were packaged by a three-plasmid system and then injected into the cochlea of P0 ICR mice, respectively; after 2-3 weeks, samples were taken for fluorescence observation and phenotype analysis of the infection of the supporting cells (see Figure 1A).

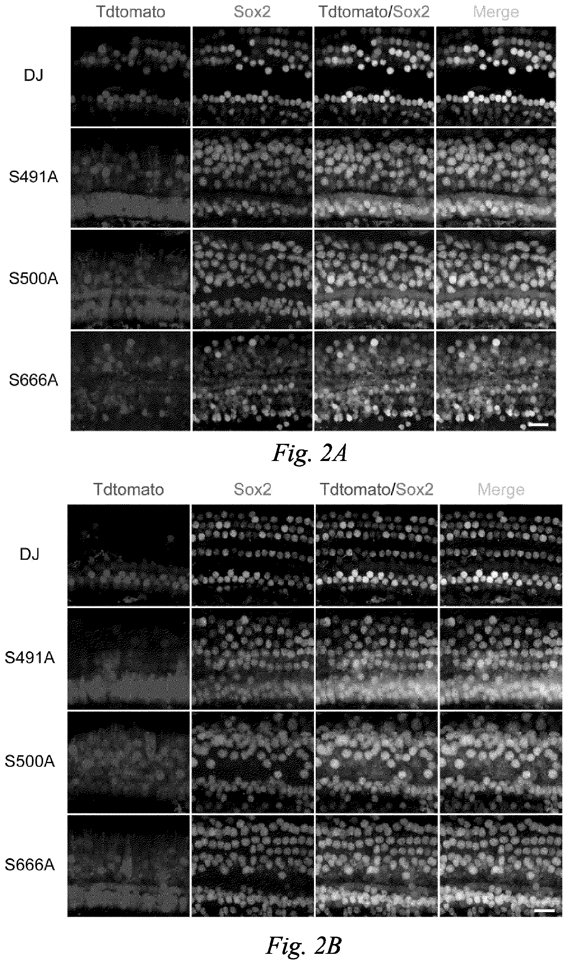

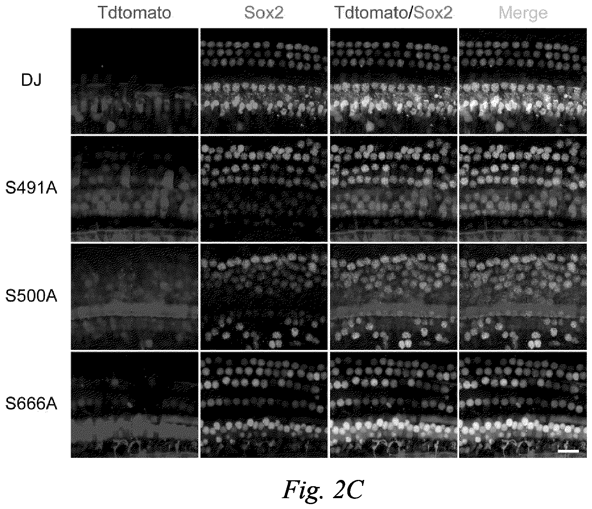

The results show that S491A, S500A, and S666A mutants could effectively improve the infection ability of DJ in supporting cells (see Figure 2), especially in basal supporting cells and middle supporting cells, so the 3 mutants were used for the subsequent experiments.

### Example 2: Infection efficiency in mouse cochlea

AAV-DJ and its mutant virus were injected into the cochlea of P0 ICR mice, respectively. Each mouse was injected with 4 × 10⁹ vg AAV virus. Similarly, 3 weeks after injection, the basal membranes of mouse cochlea were stripped and stained, and the proportions of Tdtomato positive cells in the apical, middle, and basal supporting cells were calculated, respectively.

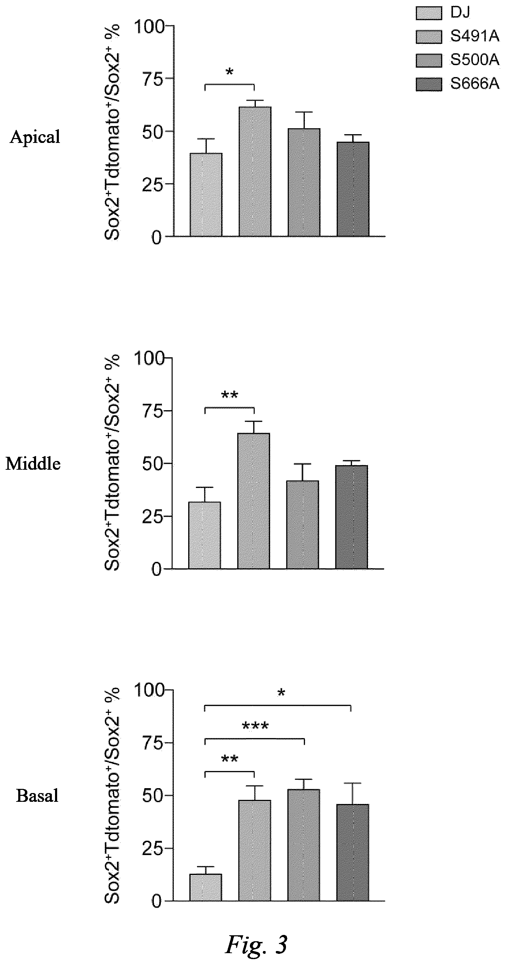

The experimental results show that for AAV-DJ mutants (S491A, S500A, and S666A), 55.7%, 51.42%, and 45.0% of the supporting cells (sox2 positive) in the apical portion were Tdtomato positive, respectively, which are higher than 39.6% for AAV-DJ, but show no significant difference (Figure 2A and Figure 3).

For AAV-DJ mutants (S491A, S500A, and S666A), 64.4%, 41.9%, and 49.2% of the supporting cells (sox2 positive) in the middle portion are Tdtomato positive, respectively, which are significantly higher than 32.0% for AAV-DJ (Figure 2B and Figure 3).

For AAV-DJ mutants (S491A, S500A, and S666A), 48.0%, 53.1%, and 45.9% of the supporting cells (sox2 positive) in the basal portion are Tdtomato positive, respectively, which are significantly higher than 12.9% for AAV-DJ (Figure 2C and Figure 3).

The infection efficiency was indicated by means of calculating the proportion of mCherry+ cells in the supporting cells in apical, middle, and basal portions within a random 100 microns visual field. The results were obtained from at least 3 mice and represented by mean ± standard deviation. *P < 0.05, **P < 0.01, ***P < 0.001, unpaired T test.

The above results indicate that in supporting cells, the mutants of AAV-DJ (S491A, S500A, and S666A) could significantly improve the infection efficiency in the supporting cells, especially the infection efficiency in basal cells.

A person skilled in the art will further appreciate that the present invention may be embodied in other specific forms without departing from the spirit or central features thereof. In that the foregoing description of the present invention discloses only exemplary embodiments thereof, it is to be understood that other variations are contemplated as being within the scope of the present invention. Accordingly, the present invention is not limited to the particular embodiments that have been described in detail herein. Rather, reference should be made to the appended claims as indicative of the scope and content of the invention.

## Claims

1. An AAV capsid protein mutant or a functional fragment thereof, **characterized in that** the AAV capsid protein mutant comprises an amino acid sequence having an amino acid mutation, as compared to a amino acid sequence of a AAV parent or wild-type capsid protein, at one or more amino acid positions corresponding to positions 491, 500, and 666 in an amino acid sequence of a parent or wild-type AAV capsid protein as set forth in SEQ ID NO: 1.

2. The AAV capsid protein mutant or functional fragment thereof according to claim 1, **characterized in that** the parent or wild-type AAV capsid protein has an amino acid sequence set forth in SEQ ID NO: 1 or an amino acid sequence having at least 85%, at least 90%, at least 91%, at least 92%, at least 93%, at least 94%, at least 95%, at least 96%, at least 97%, at least 98%, or at least 99% sequence identity thereto.

3. The AAV capsid protein mutant or functional fragment thereof according to claim 1, **characterized in that** the parent or wild-type AAV capsid protein is from AAV1, AAV2, AAV3A, AAV3B, AAV4, AAV5, AAV6, AAV7, AAV8, AAV9, AAV10, AAV11, AAV12, or AAV 13, and preferably, the parent or wild-type AAV capsid protein has an amino acid sequence selected from SEQ ID NOs: 6-19 or an amino acid sequence having at least 85%, at least 90%, at least 91%, at least 92%, at least 93%, at least 94%, at least 95%, at least 96%, at least 97%, at least 98%, or at least 99% sequence identity thereto.

4. The AAV capsid protein mutant or functional fragment thereof according to any one of claims 1-3, **characterized in that** the AAV capsid protein mutant comprises:
an amino acid sequence having an amino acid mutation, as compared to the amino acid sequence of the parent or wild-type AAV-DJ capsid protein set forth in SEQ ID NO: 1, at one or more amino acid positions corresponding to positions 491, 500, and 666 in the amino acid sequence of the parent or wild-type AAV-DJ capsid protein;
an amino acid sequence having an amino acid mutation, as compared to the amino acid sequence of the parent or wild-type AAV2 capsid protein as set forth in SEQ ID NO: 7, at one or more amino acid positions corresponding to positions 489 and 498 in the amino acid sequence of the parent or wild-type AAV2 capsid protein;
an amino acid sequence having an amino acid mutation, as compared to the amino acid sequence of the parent or wild-type AAV8 capsid protein as set forth in SEQ ID NO: 14, at one or more amino acid positions corresponding to positions 492, 501, and 667 in the amino acid sequence of the parent or wild-type AAV8 capsid protein; or
an amino acid sequence having an amino acid mutation, as compared to the amino acid sequence of the parent or wild-type AAV9 capsid protein as set forth in SEQ ID NO: 15, at one or more amino acid positions corresponding to positions 490 and 499 in the amino acid sequence of the parent or wild-type AAV9 capsid protein.

5. The AAV capsid protein mutant or functional fragment thereof according to claim 1, **characterized in that** the mutation results in the AAV capsid protein mutant having increased activity of targeting supporting cells such as apical, middle, and basal supporting cells in the inner ears of a subject.

6. The AAV capsid protein mutant or functional fragment thereof according to any one of claims 1-5, **characterized in that** the amino acid mutation is amino acid deletion or amino acid substitution.

7. The AAV capsid protein mutant or functional fragment thereof according to claim 6, **characterized in that** the amino acid substitution is a substitution with an amino acid that cannot be easily modified by phosphorylation, for example, a substitution with an acidic amino acid, a basic amino acid, a non-polar amino acid, or an aromatic amino acid except tyrosine, or a small amino acid except threonine, and preferably a substitution with alanine.

8. The AAV capsid protein mutant or functional fragment thereof according to claim 7, **characterized in** comprising an amino acid sequence having one or more amino acid substitutions selected from the group consisting of S491A, S500A, and S666A at one or more positions corresponding to positions 491, 500, and 666 in the amino acid sequence of the parent or wild-type AAV capsid protein as set forth in SEQ ID NO: 1.

9. The AAV capsid protein mutant or functional fragment thereof according to any one of claims 1-8, **characterized in that** the functional fragment is a fragment with N-terminal amino acid deletion.

10. The AAV capsid protein mutant or functional fragment thereof according to claim 9, **characterized in that** the functional fragment is a fragment with an amino acid deletion at a position corresponding to positions 1-137 or positions 1-202 in the amino acid sequence of the parent or wild type AAV-DJ capsid protein set forth in SEQ ID NO: 1.

11. The AAV capsid protein mutant or functional fragment thereof according to claim 1, **characterized in that** the AAV capsid protein mutant has any one of sequences of SEQ ID NOs: 2-4.

12. An isolated polynucleotide, **characterized in** comprising a nucleic acid sequence encoding the AAV capsid protein mutant or functional fragment thereof according to any one of claims 1-11.

13. A vector, **characterized in** comprising the isolated polynucleotide according to claim 12.

14. The vector according to claim 13, **characterized in that** the vector is selected from an expression vector, a shuttle vector, and an integrative vector.

15. A host cell, **characterized in** comprising the vector according to claim 13 or 14 or the polynucleotide according to claim 12.

16. The host cell according to claim 15, **characterized in that** the host cell is a prokaryotic cell, such as an *Escherichia coli,* or a eukaryotic cell, such as a yeast cell, a plant cell, a mammal cell, a human cell (such as an HEK293T cell).

17. A recombinant adeno-associated virus vector, **characterized in** comprising the AAV capsid protein mutant or functional fragment thereof according to any one of claims 1-11.

18. A recombinant adeno-associated virus particle, **characterized in** comprising:
(a) the AAV capsid protein mutant or functional fragment thereof according to any one of claims 1-11; and
(b) a heterologous polynucleotide encoding a heterologous gene product.

19. The recombinant adeno-associated virus particle according to claim 18, **characterized in that** the heterologous polynucleotide has an AAV ITR sequence at 5'- and 3'-end, and the ITR sequence is an intact or non-intact ITR.

20. The recombinant adeno-associated virus particle according to claim 18 or 19, **characterized in that** the heterologous gene is at least one selected from the group consisting of a hearing related gene, a related gene for gene editing, and a selectable marker gene.

21. The recombinant adeno-associated virus particle according to claim 20, **characterized in that** the hearing related gene is a gene that is expressed in the hair cells and/or supporting cells of a inner ear.

22. The recombinant adeno-associated virus particle according to claim 21, **characterized in that** the gene is at least one selected from the group consisting of *GJB2, SLC26A4, MYO6, CLDN14, ESRRB, TRPN, GJB3, MYO15A, CDH23, 12SrRNA, MYO15A, OTOF, IMC1, CDH23, TMPRSS3, POU3F4, USH2A, MYO1F, MYO7A, TRIOBP, KCNQ4, ADGRV1, PAX3, MITF, OTOA, PDZD7, TECTA, GPR98, KCNQ1,* and *Brn4.*

23. The recombinant adeno-associated virus particle according to claim 22, **characterized in that** the related gene for gene editing is selected from a coding gene of a gene editing enzyme and/or a guide RNA (gRNA) targeting a specific site.

24. The recombinant adeno-associated virus particle according to claim 23, **characterized in that** the gene editing enzyme is at least one selected from the group consisting of a meganuclease, a zinc finger nuclease, a transcription activator-like effector nuclease, a CRISPR-Cas system, a base editor, and a prime editing system.

25. The recombinant adeno-associated virus particle according to claim 19, **characterized in that** the ITR sequence can be from any AAV, preferably an ITR sequence derived from AAV2.

26. The recombinant adeno-associated virus particle according to any one of claims 18-25, **characterized in that** the heterologous polynucleotide further comprises at least one selected from the group consisting of a promoter sequence, a transcription initiation sequence, an enhancer sequence, an intron, a kozak sequence, a polyA sequence, a selection element, and a reporter gene.

27. A method for preparing the recombinant adeno-associated virus particle according to any one of claims 18-26, **characterized in** comprising operatively linking the heterologous polynucleotide encoding the heterologous gene product to the genome of an AAV vector comprising the AAV capsid protein mutant according to any one of claims 1-11.

28. A pharmaceutical composition, **characterized in** comprising the recombinant adeno-associated virus vector according to any one of claims 18-26 and a pharmaceutically acceptable carrier/excipient.

29. A method for infecting an inner ear cell of a mammal, **characterized in** comprising administering an effective amount of the recombinant adeno-associated virus particle according to any one of claims 18-26 to the mammal.

30. The method according to claim 29, **characterized in that** the inner ear cell is a supporting cell.

31. The method according to claim 29 or 30, **characterized in that** the administration is injection or infusion administration, for example, cochlea injection administration.

32. The method according to any one of claims 29-31, **characterized in that** the mammal is a human, monkey, ape, chimpanzee, cat, dog, bovine, horse, mouse, rat, or rabbit.

33. A method for alleviating, improving, or treating an ear disease such as a hearing disorder in a mammal subject, **characterized in** comprising administering the recombinant adeno-associated virus particle according to any one of claims 18-26 to the mammal subject.

34. Use of the recombinant adeno-associated virus particle according to any one of claims 18-26 for alleviating, improving, or treating an ear disease such as a hearing disorder in a mammal subject.
